# EUROPEAN PATENT APPLICATION

(11) **EP 2 851 080 A1**
(43) Date of publication of application: **25.03.2015**
(21) Application number: 13185715.3
(22) Date of filing: 24.09.2013
(51) Int. Cl.: A61K 36/81, A61K 36/63, A61P 35/00

(54) **Tomato powder-based composition**

(71) Applicant: MediaPharma S.r.l., 66013 Chieti (IT)
(72) Inventor: FOGLIANO, Vincenzo, 00166 Rome (IT); IACOBELLI, Stefano, 00198 Rome (IT); PIANTELLI, Mauro, 00167 Rome (IT)
(74) Representative: Heubeck, Christian

(57) **Abstract**

The present invention relates to a tomato powder based composition, methods for preparing it and its use.

## Description

The present invention relates to a tomato powder based composition, methods for preparing it and its use.

Prostate cancer (PCa) is the most common non-cutaneous malignant neoplasm in men in Western countries, responsible for 30,000 deaths per year in the United States (1). The number of afflicted men is increasing rapidly as the population of males over the age of 50 grows worldwide. Therefore, finding strategies for the prevention of prostate cancer occurrence and progression is a crucial medical challenge.

Findings from epidemiologic trials first raised awareness that tomatoes, particularly processed tomato products, were associated with a 30-40% reduction in PCa risk. A recent meta-analysis revealed that compared with those who consumed raw tomatoes infrequently, the relative risk (RR) for PCa in the highest quartile of tomato intake was reduced to 0.89 and to 0.81 for those consuming raw and cooked tomato products, respectively (2). Studies evaluating blood concentration of lycopene, the primary tomato carotenoid, provided supportive evidence of an inverse relationship between serum lycopene and PCa occurrence (3). In addition, intervention studies show that biomarkers related to prostate carcinogenesis may be altered by dietary intervention with tomato products (4). The majority of experimental carcinogenesis studies carried out with natural compounds derived from fruits and vegetables have adopted a "reductionist" approach examining single chemical components.

However, in clinical studies testing tomato and lycopene, only tomato supplementation was shown to reduce the serum prostate specific antigen levels (5,6). Similarly, in NMU-androgen-induced and Dunning R3327-H prostate cancer models of prostate carcinogenesis it was shown that the anti-carcinogenic effects of tomato powder could not be accounted by lycopene alone (7,8,9). Indeed, in addition to lycopene, tomatoes contain a variety of other phytochemicals (e.g. other carotenoids, polyphenols), in with possible anti-inflammatory and anticancer activities and constituents present in low amounts can also be relevant in determining the final efficacy of tomato against PCa (10,11).

There are several observations that point to tomato processing as a possible source of still unidentified contributors to the cancer chemoprevention.

First, during processing, the content of all-trans lycopene, ascorbate, and other natural antioxidants decreases as a result of thermal degradation and isomerization (12,13) with the formation of different cis lycopene isomers which are better absorbed respect to the all trans form. However, the total antioxidant activity of heat-processed tomato juice or other products may not decrease and in fact often increases (12-14).

Second, consumption of processed tomato products, rather than fresh tomatoes, was associated epidemiologically with a decreased risk of prostate cancer (15). During food processing, multiple chemical reactions occur between food components as a result of thermal and mechanical treatment, dehydration, and other physical conditions. One of the most notable and common chemical transformations is the Maillard reaction, which is mainly responsible for browning and specific flavoring of baked, roasted, and dried foods. Dried fruits and vegetables, including processed tomato powder, contain significant amounts of ketosamines, mostly in the form of non-digestible fructose-amino acid conjugates (16), which are partially absorbed into circulation but excreted unchanged.

The antitumor potential of synthetic ketosamines in melanoma, breast, and prostate cancer models has been reported (11,17,18). Three main classes of structurally defined antioxidant molecules are usually recognized in tomato products: (a) ascorbic acid (230mg/100g in fresh tomato; much less in processed tomato) and other vitamins, such as tocopherol; (b) carotenoids, including lycopene and carotene (up to 60mg/100g in fresh tomato); (c) phenolic compounds (up to 550mg/100g), including conjugates of caffeic and coumaric acids, flavonoids quercetin and kaempferol, etc. (19).

Ketosamines, with their total content of up to 8,000 mg/100 g tomato powder dry matter, represent a novel type of potential dietary antioxidants, albeit specific for foods prepared from reconstituted dried tomato products (11,20).

The last decade has seen a remarkable shift in animal-based cancer research from xenografted tumor models toward transgenic models of disease. The intellectual motivation behind this transformation is the belief that transgenic models will recapitulate the complete course of carcinogenesis more accurately (21). This hypothesis is rooted in the recognition of several advantages that transgenic models offer when compared to xenograft systems. Among these are that the process of carcinogenesis begins with normal cells, progresses through distinct genetic and histological stages, occurs in an immune-competent host, occurs in its proper cellular microenvironment, and that metastasis can occur along routes and to sites relevant to clinical disease. A perhaps unrecognized attribute lies in the fact that, because the disease is not initiated by human action but instead by a genetic program that passes through the germline, the disease process is "reset" each generation. Statistically, the progression of a transgenic model of cancer should therefore be precisely recapitulated across time and between colonies. Given appropriate record keeping and data analysis, this feature should make possible epidemiological- style investigations of great statistical power, free from the mathematical noise of genetic and environmental variation, free from many of the economic and ethical constraints of human medicine. Genetically engineered mouse (GEM) models have been utilized in cancer research to identify pathways involved in cancer development, to investigate the role of particular gene mutations/deletions in oncogenesis, and to validate key genes as therapeutic targets. These models have widely been employed to test preventive regimens, combinations of chemopreventive agents and/or drugs, cancer vaccines, and targeted therapies for the control of prostatic cancer (22).

In man, early prostate tumorigenesis appears to be associated with a dysplasia that starts with proliferative inflammatory atrophy wich may progress to low-grade Prostatic Intraepithelial Neoplasia (PIN), to high-grade PIN, to primary cancer, to metastatic cancer, and to hormone refractory cancer after hormonal treatment (23,24). Transgenic adenocarcinoma of the mouse prostate (TRAMP) is a model in which the progression of prostate cancer mirrors the stages of the human form, from PIN to undifferentiated, androgen independent tumor (25). Expression od the SV40 early genes driven by the prostate-specific promoter probasin leads to prostate cell transformation and all TRAMP mice develop prostate cancer spontaneously. This model, therefore, is regularly used to assess chemoprevention of prostate cancer (22).

Chronic inflammation/angiogenesis is also of importance in both mouse and human prostate cancer development and progression (26,27). Inflammation has been implicated as a potential aetiological agent in human prostate cancer. Viral and bacterial infections or even chemical carcinogens such as those found in cooked meat have been proposed as the inflammatory stimuli, but the mechanism of cancer induction is unknown (26). Recent information about gene expression patterns in normal and malignant epithelial stem cells from human prostate provides a new hypothesis for inflammation-induced carcinogenesis. The hypothesis states that in the stem cells located in the basal cell compartment of the prostate, activated prostate epithelial stem cells acquire a survival advantage, by expressing one of more of the same cytokines such as interleukin-6 (IL6). The establishment of one or more autocrine signaling loops results in an expansion of these cells in the absence of inflammation, as a potential first stage in the development of the tumor (28). IL-6-type cytokine (IL-6, oncostatin M, and IL-11) and their receptors are expressed in the prostate and regulate its growth in an autocrine and paracrine manner (see ref. 32 for review). Because serum IL-6 and IL-11 concentrations increase in patients with metastatic and hormone-refractory prostate cancer (33), measurement of IL-6 concentrations leads to more accurate prediction of disease progression and survival. In addition, IL-6 plays an important role in the transition from an androgen-dependent to an androgen independent state, promotes neuroendocrine differentiation, and contributes to cachexia in prostate cancer patients (34,35).

Vascular Endothelial Growth Factor (VEGF) is highly correlated with angiogenesis and metastasis formation in both men and TRAMP mice (29-31). It was inhibited by TD, which delayed/blocked the angiogenic switches needed for the development, first of P and then of poorly differentiated, hormone independent tumors (30).

In a recent paper (36), we reported that a tomato diet (*Control Diet enriched with 10% of a "tomato powder", whose composition is reported in Table 1*), increased TRAMP mice survival. Actually, Kaplan-Meier survival curves revealed a significant increase in the overall survival rate of animals fed with the Tomato Diet mice when compared to animals fed with Control Diet (67% versus 11 %; P = 0.0018) (36).

The survival proportion of Lycopene treated mice (17%) was not significantly different from that of mice receiving Control Diet (11%) (P = 0.384) (unpublished observations).

Again, the tomato diet delayed progression from prostatic intraepithelial neoplasia to adenocarcinoma, and decreased the incidence of poorly differentiated carcinoma in TRAMP mice. Biochemical data disclosed an increase of serum antioxidant activity and a reduction of serum level of inflammation/angiogenesis biomarkers of particular relevance for prostate carcinogenesis (36), such as VEGF and IL6.

In view of the above, it was the object of the present invention to provide a novel tomato-based composition which is even more active in particular more active in reducing VEGF and IL6 serum levels in a patient.

This problem has been solved providing a composition comprising a first component and a second component wherein:
(a) the first component is a tomato powder; and
(b) the second component is a polyphenolic extract, preferably derived from olive oil and/or olive oil waste waster.

According to an especially preferred embodiment, the tomato powder and/or the polyphenolic extract are prepared as herein described below.

Preferably the tomato powder is enriched in carotenoids, flavonoids and/or ketosamines in particular if compared with tomato diet described above.

For example, the tomato powder may comprise carotenoids, such as all-trans lycopene, 5-cis lycopene, lutein and/or β-carotene, in an amount of about 0.40-0.60 % weight (weight/weight) (w/w), more preferably of about 0.45-0.55 % w/w and most preferably of about 0.50 % w/w.

The tomato powder further preferably comprises flavonoids, such as quercentin, naringenin and/or derivatives thereof, in amount of about 0.10-0.30 % w/w, more preferably about 0.15-0.25 % w/w and most preferably of about 0.20 % w/w.

Ketosamines are preferably comprised in the tomato powder in an amount of 0.006-0.01 % w/w, more preferably about 0.008 % w/w. An example for especially preferred ketosamines are fructosyl-amino acids and in particular Fru-His. Fru-His represent a potential dietary antioxidant. The relatively high affinity of Fru-His to copper at physiological pH provides for very low residual free copper in the presence of Fru-His and thus can translate into inactivation of this redox catalyst and protection of biologically important molecules from oxidative degradation in vivo. Fru-His demonstrates the potential to efficiently block metal-dependent oxidative damage of biopolymers but may also exhibit the ROS scavenging capabilities. In a particular preferred embodiment, the tomato powder used according to the present invention comprises Fru-His in an amount of 0.00005-0.00007 % w/w and even more preferably in an amount of about 0.00006 % w/w.

According to a preferred embodiment, the tomato powder comprises by weight about 0.5 % carotenoids, about 0.2 % flavonoids, about 0.008 % ketosamines. Further proteins, carbohydrates like saccharides, lipids and/or alimentary fibers may also be comprised. Also α-tocopherol may be comprised by the tomato powder described herein.

According to a especially preferred embodiment, the tomato powder is a dry extract, i.e. contains less less than about 6 % w/w humidity, more preferably less than 5 % w/w humidity and even more preferably less than 4 % w/w humidity and most preferably less than 3 % w/w humidity.

According to an especially preferred embodiment, the tomato powder comprises by weight about 0.5 % carotenoids, about 0.2 % flavonoids, about 0.008 % ketosamines, about 15-18 %, more preferably 16.5 % proteins, about 60-70 %, more preferably about 63.5 % carbohydrates, about 2-5 %, more preferably about 3.4 % lipids, and/or about 14-20 %, more preferably about 15.9 % dietary fibers and less than about 4 % humidity. In other words a 100 g sample of tomato powder according to the invention comprises about 96 g or more dry matter, about 63,5 g carbohydrates, about 3.4 g lipids, about 500 mg carotenoids, about 190 mg lycopene isomers, about 2.3 mg α-tocopherol, about 200 mg flavonoids, about 8 mg ketosamines and/or about 15.9 g fibers.

As already stated above, according to an preferred embodiment, the phenolic extract used in the composition according to the present invention, is prepared as herein described below. The polyphenolic extract is preferably an extract of waste water of olive oil production. Processes of suitable olive oil production ways are known to the person skilled in the art.

According to an especially preferred embodiment, the polyphenolic extract comprises
about 5-7 % w/w, more preferably about 6 % w/w oleuropeinaglycon, about 1-3 % w/w, more preferably about 2 % w/w ligstrosideaglycon,
about 15-17% w/w, more preferably about 6 % w/w
oleuropeindialdehydeaglycon,
about 6-8 % w/w, more preferably about 7 % w/w ligtrosidealdehydeaglycon, about 5-7 % w/w, more preferably about 6 % w/w verbascoside,
about 4-6 % w/w, more preferably about 5 % w/w pinoresinol and deacetoxy-pinoresinol,
about 2-4 % w/w, more preferably about 3 % w/w tyrosol,
about 9-11 % w/w, more preferably about 10 % w/w hydroxytyrosol,
about 7-9 % w/w, more preferably about 8% w/w unidentified polyphenols, and/or
about 32-34 % w/w, more preferably about 33 % w/w polysaccharides and less than about 4 % w/w humidity.

According to further preferred embodiment, the composition characterized by a weight ratio of the first component, i.e. the tomato powder, and the second component, i.e. the polyphenolic extract, of about 100-95 : 0-5, more preferably 99-96 : 1-4, even more preferably 99-98 : 1-2, in particular preferably about 98 : 2 and most preferably about 99.8 : 0.2.

The inventors were able to show, that a composition as presented and claimed herein significantly reduces serum levels of the proangiogenic/proinflammatory mediators VEGF and IL6. Surprisingly, the combination of a tomato powder as described herein with a polyphenolic extract as described herein even leads to reduced VEGF and/or IL6 levels in serum if compared to a tomato diet as known from the prior art (36). Therefore, the present invention also relates to the use of an inventive composition in a method for reducing proangiogenic/pro-inflammatory mediators, such as VEGF and IL6. VEGF and IL6 are involved in carciogenesis and tumor progression, in particular prostate carciogenesis and tumor progression.

Thus, according to a further aspect of the present invention, the inventive composition is for use in a method or prevention and/or treatment of cancer or inflammation. The inventive composition is able to counteract the inflammation of the prostate gland and to prevent prostate cancer onset and/or progression. The cancer may be selected from the group of prostate cancer, colon cancer, breast cancer, lung cancer, renal cancer, liver cancer and/or metastasis thereof. In an especially preferred embodiment, the cancer is prostate cancer.

According to a further preferred embodiment, the present invention relates to a composition for use in a method for prevention of cancer, particularly prostate cancer.

A further aspect of the present invention relates to a composition described herein for use as a food supplement. Accordingly, the present invention also relates to the use of a composition described herein as food supplement.

The composition for use in a method according to the present invention can be present in the form of a pharmaceutical composition and/or formulation. Such a composition and/or formulation according to the invention can be administered to a subject in need thereof, particularly a human patient, in a sufficient dose for the prevention and/or treatment of the specific conditions by suitable means. For example, the composition and/or formulation according to the invention may be formulated as composition together with acceptable carriers, preservatives, stabilisers, diluents and/or adjuvants. Such agents are known to the person skilled in the art. Therapeutic efficiency may be determined according to standard protocols.

The composition or formulation may be administered which seems suitable to the person skilled in the art. However, preferably the composition described herein is to be administered orally. For example the composition may be administered in form of a water soluble powder or tablets, capsules, caplets.

The dose of the composition and/or formulation administered will, of course, be dependent on the subject to be treated and on the condition of the subject such as the subject's weight, the subject's age and the type and severity of the disease or injury to be treated, the manner of administration and the judgement of the prescribing physician. For example, a daily dose of 0.001 to 100 mg/kg is suitable.

A further aspect of the present invention relates to a method for the production of a tomato powder which is preferably to be used in the inventive composition comprising the steps:
(a) providing a tomato puree preferably having an increased content of tomato seeds and skins;
(b) concentrating the tomato puree from (a) at about 50-70 °C, preferably about 60 °C under a pressure in a range between about 300 and 400 mbar obtaining a tomato concentrate;
(c) spray drying the tomato concentrate from (b), preferably using an inlet temperature in a range between about 170-200 °C, preferably about 175-190 °C, to obtain a tomato powder.

The tomato puree provided in step (a) can be obtained by washing, sorting and washing tomatoes plum avoiding a refining step, thereby increasing the presence of seeds and skins rich of flavonoids.

The tomato powder obtained is strongly enriched in bioactive compounds such as carotenoids, flavonoids and/or ketosamines. Of course, further aspect of the present invention is a tomato powder prepared by the method described above.

Moreover, a method for the production of a polyphenolic extract, preferably to be used in the inventive composition, is described herein, comprising the steps:
(a) filtrating waste wasters from olive oil production through at least one filter;
(b) concentrating the retentate obtained from (a) under reduced pressure at a temperature up to 20 °C, preferably up to a concentration of 10-15 % w/w dry matter;
(c) spray drying the concentrated retentate from (b) in the presence of hydrocolloids using an inlet temperature of about 150-170 °C, preferably about 170° C and an outlet temperature below 80 °C.

The waste waters from olive oil production used in step (a) may be first mechanically filtered to avoid big particles and/or be treated with pectinolytic enzymes. Thereafter a microfiltration step is carried out which may comprise the use of several different membranes such as polymeric and/or ceramic membranes. For example, the microfiltration can be carried out on polymeric membrane with porosity of 25000 Da and the permeate obtained may be passed through a membrane with a cut off of 8000 Da. The retentate obtained can be concentrated by several passages on the same membrane.

Step (c) spray drying may be done in the presence of hydrocolloids, such as two hydrocolloids (e.g. acacia gum and maltodextrin), for example, at 50% each. The ratio between hydrocolloids acting as coating agent and the core can be between 60:40 to 50:50.

Therefore, another object of the present invention is a polyphenolic extract as prepared by the method described above.

A further aspect of the present invention relates to method for the production of the inventive composition combining the steps for preparing a tomato powder and a polyphenolic extract as described above:
(a) providing a tomato puree preferably having an increased content of tomato seeds and skins;
(b) concentrating the tomato puree from (a) at about 50-70 °C, preferably about 60 °C under a pressure in a range between about 300 and 400 mbar obtaining a tomato concentrate;
(c) spray drying the tomato concentrate from (b), preferably using an inlet temperature in a range between about 170-200 °C to obtain a tomato powder; and
(d) mixing the tomato powder from (c) with a polyphenolic extract from olive oil waste water which may be obtained as herein described.

### Figures:

**Figure 1****:** Carotenoid recovery as a function of time/temperature conditions during processing.
**Figure 2****:** Flavonoids recovery as a function of time/temperature conditions during processing
**Figure 3****:** Fructosyl-amino acids recovery as a function of time/temperature conditions during processing.

Initial condensation of free amino acids with glucose in dried vegetables, fruits, and other food products brings about formation of Fructosyl-amino acids, also known as Amadori rearrangement products, which may constitute up to 12% of the water-solubles per dry weight. As shown, the processing conditions of the A Component maximize the presence of fructosyl-amino acids and particularly of Fru-HIS.

Ingested D-fructose-amino acids resist digestion and can be partially absorbed into circulation, without being metabolized. Given that Fru-His is present in significant amount in RED007, the data presented suggest that FruHis plays a key role in the pool of active compounds responsible for RED007 physiological effects.

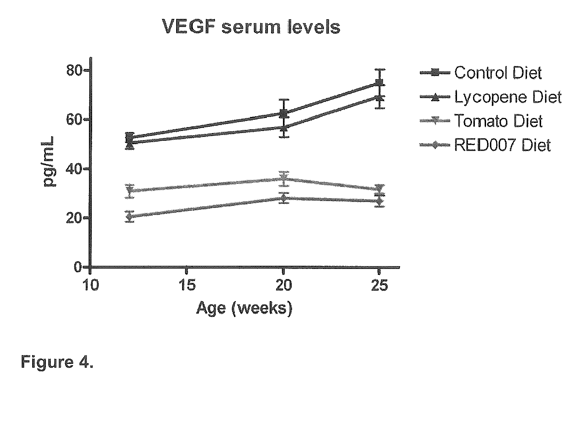

**Figure 4****:** VEGF serum levels during different diets. Values represent mean ± SD (n 8 mice). **RED007 diet** *(CD diet fortified with 10% of the Final Product* **RED007**), when compared to **Control Diet,** significantly reduced serum levels of VEGF: P < 0.0001 (two-ways ANOVA). More importantly, **RED007 diet is** significantly more efficient than **Tomato Diet** (*Control Diet enriched with 10% of the "tomato powder", whose composition is reported in Table 1)* in reducing serum VEGF concentrations (P<0.001). **Lycopene Diet** did not significantly modify the VEGF concentrations found in mice feed with Control Diet (P = 0.204).

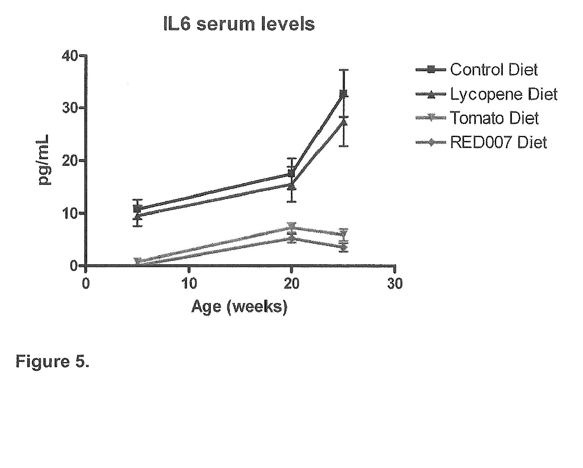

**Figure** 5: IL6 serum levels during different diets. Values represent mean ± SD (n 8 mice). **RED007 diet** *(CD diet fortified with 10% of the Final Product* **RED007**), significantly reduced serum levels of IL6: P < 0.0001 (two-ways ANOVA). More importantly, **RED007 diet is** significantly more efficient than **Tomato Diet** (*Control Diet enriched with 10% of the "tomato powder", whose composition is reported in Table 1*) in reducing serum VEGF concentrations (P<0.001). **Lycopene Diet** did not significantly modify IL6 concentrations (P = 0.314).

### Examples

### Preparation of tomato powder (Component A)

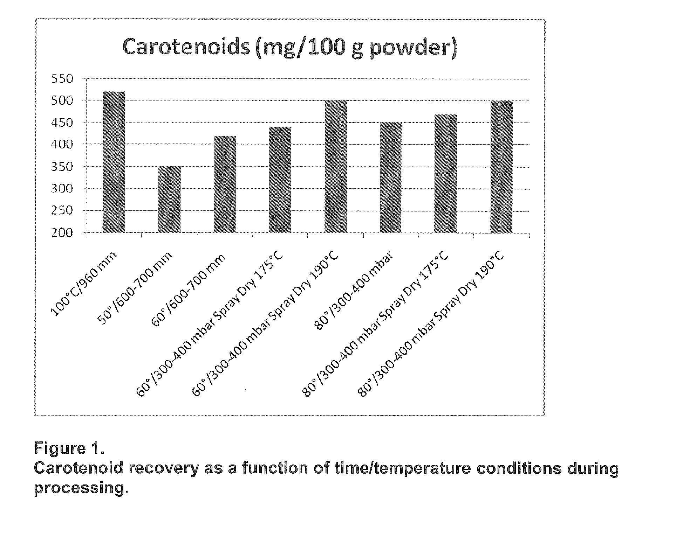

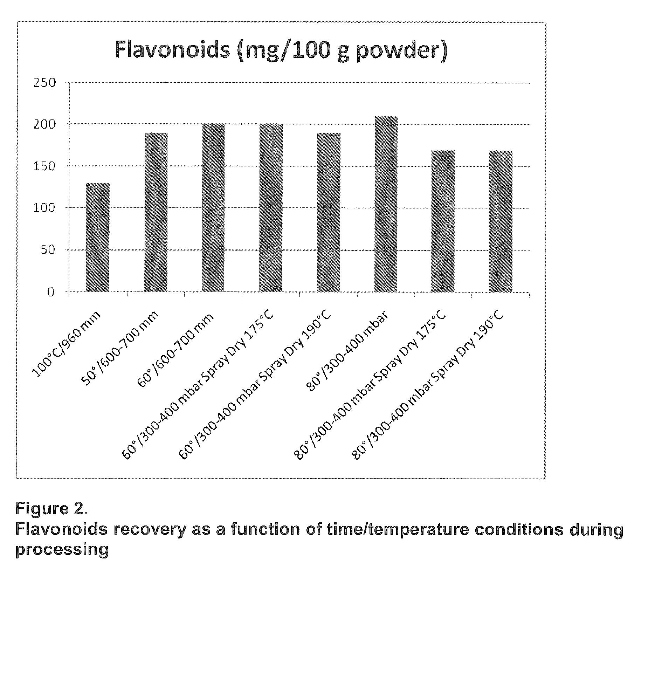

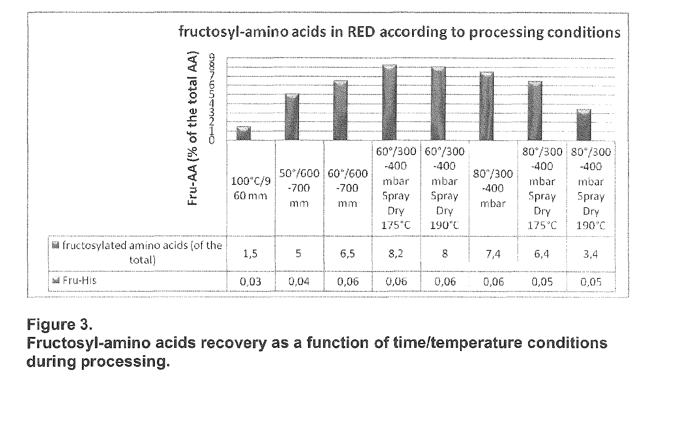

To prepare the tomato powder the tomatoes plum were firstly washed and sorted and then were crushed avoiding the refining step to increase the presence of seeds and skins rich of flavonoids. The tomato puree obtained was pre heated at temperature between 80-90°C, and then concentrated using low temperature (60°C) and vacuum (300-400 mbar). When the refractive index of juice was around 29-30 Brix grade it was diluted again with hot water at a concentration 12 Brix grades and spray dried using a inlet temperature 190°C and the outlet temperature was kept below 85 °C. Turbine speed was of 2600 rpm. These condition have been chosen on the basis of preliminary experiments aimed at optimizing the final content in Carotenoids, Flavonoids and Fructosyl-amino acids (and particularly Fru-His). Figure 1-3.

As a consequence, when compared to the composition of the tomato powder already prepared for chemoprevention experiments in mice (36,38), the A component,i.e. tomato powder here described is strongly enriched in active compounds (Table 1). It should be noted that a conventional tomato paste was completely ineffective in the TRAMP prostate cancer model (38).

**TABLE I. Composition for 100 g:**

| | **Tomato powder*** | **A Component (Claim 1)** |
|---|---|---|
| **Dry matter** | **≥ 95%** | **96%** |
| **Carbohydrates (g)** | **66** | **63.5** |
| **Proteins (g)** | **10.2** | **16.5** |
| **Lipids (g)** | **1.2** | **3.4** |
| **Total carotenoids (mg)** | **142.2** | **500** |
| *All-trans lycopene* | *109.2* | *250* |
| *5-cis lycopene* | *7.4* | *35* |
| **Lycopene *isomers*** | *15.7* | *190* |
| b*-carotene* | *8.7* | *22* |
| *Lutein* | *1.2* | *3* |
| a**-tocopherol (mg)** | **1.9** | **2.3** |
| **Total flavonoids (mg)** | **15.3** | **200** |
| *Quercetin deriv.* | *1.1* | *140* |
| *Naringenin deriv.* | *4.2* | *60* |
| **Ketosamines (mg)** | **ND** | **8** |
| *Fru*/*HIS* | *ND* | *0.06* |
| **Fibers** | **ND** | **15.9** |

| | | |
|---|---|---|
| *Tomato preparation used in the experiments reported in ref. 36 and in Fig. 4 and 5. ND= not determined | | |

The concentration of carotenoids and flavonoids was determined by HPLC using a C30 and a C18 chromatographic columns coupled with and UV-Vis detection as previously described. The concentration of FRU-HIS was determined by High Resolution Mass Spectrometry (HRMS) using an ExactiveOrbitrap equipment (ThermoFisher, USA).

### Preparation of polyphenolic extract (Component B) from olive oil wastewater

Olive polyphenols were obtained from the Olive Oil Mill Wastewater of a Coratina variety. Waste waters were subjected to a mechanical filtering process (100µm of cut off) to avoid big particle entering and pluging the lower cut off filtration unit. After that they were enzymatically treated with a pectinolytic enzymes before filtrating them in several filtration step. The microfiltration was carried out on polymeric membrane with porosity of 25000D and the permeate was passed through membrane with a cut off of 8000 Da. The retentate by this membrane was concentrated with several passage on the same membrane and after a light evaporation under reduced pressure without exceeding 20 °C up to a concentration of 10-15% of dry matter. This fraction was spray dried using in the presence of two hydrocolloids (acacia gum and maltodextrin) at 50% each. The ratio between hydrocolloids acting as coating agent and the core was between 60:40 to 50:50. Spray drying conditions were as follow: inlet temperature 160 °C and the outlet Temperature was kept below 80°C with a final product moisture not more than 7%. Turbine speed was of 2700 rpm.

**TABLE II. B Component: composition for 100 g**

| | |
|---|---|
| **oleuropeinaglycon** | **6 g** |
| **ligtrosideaglycon** | **2 g** |
| **oleuropeindialdehydeaglycon** | **16 g** |
| **ligtrosidedialdehydeaglycon** | **7 g** |
| **verbascoside** | **6 g** |
| **pinoresinol and deacetoxy-pinoresinol** | **5 g** |
| **thyrosol** | **3 g** |
| **hydroxy-thyrosol** | **10 g** |
| **unidentified polyphenols,** | **8 g** |
| **polysaccharides** | **33 g** |
| **humidity** | **<4 g** |

*Detailed description of the methods obtaining the data presented in* *figures 1 to 3**.*

To 3.0 g of tomato powder or 9.0 g of tomato paste, 20 mL of distilled water were added. After mixing for 30 min, aliquots were centrifuged at 5.000 rpm for 20 min at 4 °C, and the clear supernatant was subjected to centrifugal ultrafiltration using membrane with a cut off of 3 KDa (Millipore, USA) for 2 h at 4 °C. The resulting filtrate was then freeze-dried for further analysis. Extractions were performed on the tomato preparation for RED prepared using different time/temperature of processing.

### Preparation of a composition of the invention

A composition according to the invention, i.e. a composition comprising tomato powder and polyphenolic extract, may herein also be called RED007.

Component A, i.e tomato powder and component B, i.e. polyphenolic extract were mixed in a ratio 98:2 using a powder mixer under controlled humidity conditions and immediately packed under vacuum.

RED007 significantly reduces serum levels of the proangiogenic/ proinflammatory mediators VEGF and IL6 involved in prostate carcinogenesis and tumor progression. As reported in Figure 4 and 5, (unlike lycopene) RED007 significantly reduces serum levels of proangiogenic/ proinflammatory mediators VEGF and IL6 in TRAMP mice.

### REFERENCES

1) Jema A, Murray T, Ward E, et al. Cancer statistics, 2005. CA Cancer J Clin 2005;55:10-30.
2) Etminan M, Takkouche B, Caamano-Isorna F. The role of tomato products and lycopene in the prevention of prostate cancer: a meta-analysis of observational studies. Cancer Epidemiol Biomarkers Prev 2004;13:340-5.
3) Wu K, Erdman JW, Jr., Schwartz SJ, et al. Plasma and dietary carotenoids, and the risk of prostate cancer: a nested case-control study. Cancer Epidemiol Biomarkers Prev 2004;13:260-9.
4) Gupta S. Prostate cancer chemoprevention: current status and future prospects. ToxicolApplPharmacol 2007;224:369-76.
5) Chen L, Stacewicz-Sapuntzakis M, Duncan C, et al. Oxidative DNA damage in prostate cancer patients consuming tomato sauce-basedentrees as a whole-food intervention. J Natl Cancer Inst 2001;93: 1872-9.
6) Kucuk O, Sarkar FH, Djuric Z, et al. Effects of lycopene supplementation in patients with localized prostate cancer. ExpBiol Med (Maywood) 2002;227:881-5.
7) Imaida K, Tamano S, Kato K, et al. Lack of chemopreventive effects of lycopene and curcumin on experimental rat prostate carcinogenesis. Carcinogenesis 2001;22:467-72.
8) Boileau TW, Liao Z, Kim S, Lemeshow S, Erdman JW, Jr., Clinton SK. Prostate carcinogenesis in N-methyl-N-nitrosourea (NMU)-testosterone- treated rats fed tomato powder, lycopene, or energy-restricted diets. J Nat Cancer Inst 2003;95:1578-86.
9) Canene-Adams K, Lindshield BL, Wang S, et al. Combinations of tomato and broccoli enhance antitumor activity in Dunning R3327-H prostate adenocarcinomas. Cancer Res 2007;67:836-43.
10) U.S. Department of Agriculture National Nutrient Database for Standard Reference: release 19 (2006), http://www.nal.usda.gov/fnic/foodcomp/cgi-bin/measure.pl? MSRE_NO=11548xyz1100 xyzTomato%20powderxyzxyz
11) Mossine VV, Chopra P, Mawhinney TP. Interaction of tomato lycopene and ketosamine against rat prostate tumorigenesis. Cancer Res 2008;68:4384-91.
12) Nikoli MC, Anese M, Parpinel MT, Francesci S, Lerici CR. Loss and/or formation of antioxidants during food processing and storage. Cancer Lett 1997;114:71-4.
13) Gahler S, Otto K, Bo"hm V. Alterations of vitamin C, total phenolics, and antioxidant capacity as affected by processing tomatoes to different products. J Agric Food Chem 2003;51:7962-8.
14) Dewanto V, Wu X, Adom KK, Liu RH. Thermal processing enhances the nutritional value of tomatoes by increasing total antioxidant capacity. J Agric Food Chem 2002;50:3010-4.
15) Giovannucci E, Clinton SK. Tomatoes, lycopene, and prostate cancer. ProcSocExpBiol Med 1998;218:129-39.
16) Eichner K, Reutter M, Wittmann R. Detection of Maillard reaction intermediates by high pressure liquid chromatography (HPLC) and gas chromatography. In: Finot PA, Aeschbacher HU, Hurrell RF, Liardon R, editors. The Maillard reaction in food processing, human nutrition and physiology. Basel (Switzerland): BirkhäuserVerlag; 1990. p. 63-77.
17) Glinsky GV, Price JE, Glinsky VV, Mossine VV, Kiriakova G, Metcalf JB. Inhibition of human breast cancer metastasis in nude mice by synthetic glycoamines. Cancer Res 1996;56:5319-24.
18) Glinsky VV, Glinsky GV, Glinskii OV, et al. Intravascular metastatic cancer cell homotypic aggregation at the sites of primary attachment to the endothelium. Cancer Res 2003;63:3805-1.
19) Vinson JA, Hao Y, Su X, Zubik L. Phenol antioxidant quantity and quality in foods: vegetables. J Agric Food Chem 1998;46:3630-4.
20) Eichner K, Reutter M, Wittmann R. Detection of Maillard reaction intermediates by high pressure liquid chromatography (HPLC) and gas chromatography. In: Finot PA, Aeschbacher HU, Hurrell RF, Liardon R, editors. The Maillard reaction in food processing, human nutrition and physiology. Basel (Switzerland): BirkhäuserVerlag; 1990. p. 63-77.
21) Jackson-Grusby L. Modeling cancer in mice. Oncogene 2002, 21:5504-5514
22) Lattanzio R, Lamolinara A, Piantelli M, lezzi M. Natural compounds, antioxidant and antiandrogens in the prevention of prostate cancer: In vivo evidences from murine models and human clinical studies.Chapter 16 in: Advances in Prostate Cancer, pp377-400; Hamilton G Editor. InTech, Chapters Publisher.2013 http://www.intechopen.com/books/advances-in-prostate-cancer
23) Nelson WG et al. Prostate cancer. New Engl J Medicine 2003;349:366-381.
24) Dasgupta S et al. Oncogenic activation in prostate cancer progression and metastasis : Molecular inights and future challenges. J Carcinogenesis 2012, 11:4.
25) Greenberg NM, DeMayo F, Finegold MJ, et al. Prostate cancer in a transgenic mouse. ProcNatlAcadSci USA 1995;92:3439-43.
26) De Marzo AM, Platz EA, Sutcliffe S, et al. Inflammation in prostate carcinogenesis. Nature 2007;7:256-269.
27) Charlesworth PJ, Harris AL. Mechanisms of disease: angiogenesis in urologic malignancies. Nat ClinPractUrol 2006;3:157-69.
28) Maitland NJ and Collins AT. Inflammation as the primary aetiological agent of human prostate cancer: a stem cell connection? J. Cell. Biochem. 2008;105: 931-939.
29) Delongchamps NB, Peyromaure M, Dinh-Xuan AT. Role of vascularendothelial growth factor in prostate cancer. Urology 2006;68:244-8.
30) Huss WJ, Hanrahan CF, Barrios RJ, Simons JW, Greenberg NM.Angiogenesis and prostate cancer: identification of a molecular progression switch. Cancer Res 2001;61:2736-43.
31) Isayeva T, Chanda D, Kallman L, Eltoum IE, Ponnazhagan S. Effectsof sustained antiangiogenic therapy in multistage prostate cancer in TRAMP model. Cancer Res 2007;67:5789-97.
32) Culig Z, Steiner H, Bartsch G, Hobisch A. Interleukin-6 regulation of prostate cancer cell growth. J Cell Biochem 2005;95:497-505.
33) Furuya Y, Nishio R, Junicho A, Nagakawa O, Fuse H. Serum interleukin-11 in patients with benign prostatic hyperplasia and prostate cancer. IntUrolNephrol 2005;37:69-71.
34) Wallner L, Dai J, Escara-Wilke J, et al. Inhibition of interleukin-6 with CNTO328, an anti-interleukin-6 monoclonal antibody, inhibits conversion of androgen-dependent prostate cancer to an androgenindependent phenotype in orchiectomized mice. Cancer Res 2006; 66:3087-95.
35) Kuroda K, Nakashima J, Kanao K, et al. Interleukin 6 is associated with cachexia in patients with prostate cancer. Urology 2007;69:113-7.
36) Pannellini T, lezzi M, Liberatore M, Sabatini F, lacobelliS, Cosmo Rossi C, Alberti S, Di Ilio C, Vitaglione P, Fogliano V, Piantelli M. A Dietary Tomato SupplementPreventsProstateCancer in TRAMP Mice. Cancer Prev Res 2010;3:1284-91
37) Canene-Adams K, Campbell JK, Zaripheh S, Jeffery EH, Erdman JW, Jr. The tomato as a functional food. J Nutr 2005;135:1226-30.
38) Konijeti R, Henning S, Moro A,Sheikh A, Elashoff D, Shapiro A, Ku M,Jonathan W. Said JW, Heber D, Cohen P,Aronson WJ. Chemoprevention of prostate cancer with lycopene in the TRAMP model. The Prostate 2010;70:1547-1554.

## Claims

1. Composition comprising a first component and a second component, wherein:
(a) the first component is a tomato powder; and
(b) the second component is a polyphenolic extract.

2. The composition according to claim 1 for use in a method for the prevention and/or treatment of cancer and/or the treatment of inflammation, wherein the cancer is preferably a prostate cancer.

3. The composition according to claim 1 for use as a food supplement.

4. The composition according to any of claims 1-3, wherein the weight ratio of the content of the first component to the content of the second component is about 100-95:0-5, preferably about 99,8:0,2.

5. The composition according to claims 1-6, wherein the tomato powder comprises carotenoids in an amount by weight of about 0.45-0.55 %, preferably about 0.5%, flavonoids in an amount by weight of about 0.15-0.25 %, preferably about 0.2%, ketosamines in an amount by weight of about 0.007-0.009 %, preferably about 0.008 %, preferably including Fru-His in an amount of about 0.00005-0.00007 %, even more preferably about 0.00006 %, about 16.5% proteins, about 63,5% carbohydrates, about 3.4% lipids and/or about 15,9% dietary fibers and less than about 4% humidity.

6. The composition according to any of claims 1-5, wherein the polyphenolic extract is an extract of waste water of olive oil production, wherein the polyphenolic extract comprises by weight about 5-7 %, more preferably about 6 % oleuropeinaglycon,
about 1-3 %, more preferably about 2 % ligstrosideaglycon,
about 15-17%, more preferably about 6 % oleuropeindialdehydeaglycon,
about 6-8 %, more preferably about 7 % ligtrosidealdehydeaglycon, about 5-7 %, more preferably about 6 % verbascoside,
about 4-6 %, more preferably about 5 % pinoresinol and deacetoxy-pinoresinol,
about 2-4 %, more preferably about 3 % tyrosol,
about 9-11 %, more preferably about 10 % hydroxytyrosol,
about 7-9 %, more preferably about 8% unidentified polyphenols, and/or
about 32-34 %, more preferably about 33 % polysaccharides and less than about 4 % humidity.

7. Use of a composition according to claims 1-6 in the prevention of cancer and/or the treatment of inflammation, wherein the cancer is preferably a prostate cancer and wherein the composition is preferably to be administered orally.

8. The use of a composition according to any of claims 7, wherein the administration of the composition reduces VEGF and IL6 serum levels in a patient.

9. Use of a composition according to any of claims 1, 4-6 as food supplement.

10. Method for the production of a composition according to claims 1-12, which comprises the steps:
(i) producing a tomato powder by:
(a) providing a tomato puree preferably having an increased content of tomato seeds and skins;
(b) concentrating the tomato puree from (a) at about 50-70 °C, preferably about 60 °C under a pressure in a range between about 300 and 400 mbar obtaining a tomato concentrate;
(c) spray drying the tomato concentrate from (b), preferably using an inlet temperature in a range between about 170-200 °C to obtain a tomato powder, wherein the spray drying step is preferably performed at about 190 °C; and/or
(ii) producing a polyphenolic olive extract by :
(a) filtrating waste wasters from olive oil production through at least one filter;
(b) concentrating the retentate obtained from (a) under reduced pressure at a temperature up to 20 °C, preferably up to a concentration of 10-15 % w/w dry matter;
(c) spray drying the concentrated retentate from (b) preferably in the presence of hydrocolloids using an inlet temperature of about 150-170 °C, preferably about 170° C and an outlet temperature below 80 °C.
(iii) mixing the tomato powder from (i) and the polyphenolic olive extract from (ii) in a weight ratio of about 100-95:0-5, preferably about 99,8:0,2.

11. Tomato powder prepared by the method of claim 10.

12. Polyphenolic extract prepared by the method of claim 10.

13. Tomato powder comprising by weight about 0.5% carotenoids, about 0.2% flavonoids, about 0.008% ketosamines, about 16.5% proteins, about 63,5% carbohydrates, about 3.4% lipids, about 15,9% alimentary fibers and less than about 4% humidity.

14. Polyphenolic extract comprising by weight
about 5-7 %, more preferably about 6 % oleuropeinaglycon,
about 1-3 %, more preferably about 2 % ligstrosideaglycon,
about 15-17 %, more preferably about 6 % oleuropeindialdehydeaglycon,
about 6-8 %, more preferably about 7 % ligtrosidealdehydeaglycon, about 5-7 %, more preferably about 6 % verbascoside,
about 4-6 %, more preferably about 5 % pinoresinol and deacetoxy-pinoresinol,
about 2-4 %, more preferably about 3 % tyrosol,
about 9-11 %, more preferably about 10 % hydroxytyrosol,
about 7-9 %, more preferably about 8% unidentified polyphenols, and/or
about 32-34 %, more preferably about 33 % polysaccharides and less than about 4 % humidity.
